Europäisches Patentamt

European Patent Office

Office européen des brevets

(11) Publication number: **0 500 672 B1**

# EUROPEAN PATENT SPECIFICATION

(45) Date of publication of patent specification: **09.08.95**　(51) Int. Cl.6: **A61F 4/00**, G06F 3/037, //H04N7/18

(21) Application number: **90916833.8**

(22) Date of filing: **06.11.90**

(86) International application number:
**PCT/SE90/00718**

(87) International publication number:
**WO 91/06263 (16.05.91 91/11)**

(54) **A COMMUNICATION DEVICE.**

(30) Priority: **07.11.89 SE 8903713**

(43) Date of publication of application:
**02.09.92 Bulletin 92/36**

(45) Publication of the grant of the patent:
**09.08.95 Bulletin 95/32**

(84) Designated Contracting States:
**AT BE CH DE DK ES FR GB IT LI LU NL SE**

(56) References cited:
**WO-A-86/01963**
**WO-A-87/07497**

(73) Proprietor: **PALSGARD, Göte**
**Nygatan 28**
**S-702 11 Örebro (SE)**

Proprietor: **ÖSTLIN, Lars O.**
**Gundbo 3328**
**S-822 00 Alfta (SE)**

(72) Inventor: **PALSGARD, Göte**
**Nygatan 28**
**S-702 11 Örebro (SE)**
Inventor: **ÖSTLIN, Lars O.**
**Gundbo 3328**
**S-822 00 Alfta (SE)**

(74) Representative: **Bjerkén, Jarl Hakan**
**Bjerkéns Patentbyra KB**
**P.O.Box 1274**
**S-801 37 Gävle (SE)**

Rank Xerox (UK) Business Services
(3.10/3.09/3.3.3)

## Description

### FIELD OF THE INVENTION AND PRIOR ART

The present invention relates to a device for making it possible for in particular disabled persons without capability to speak and to move their arms to communicate with the environment according to the preamble of claim 1. A device of this type is known by our WO 87/07497 (SE-A-453 959) and remedies the inconveniences of already known devices for communication with the environment by determination of the gaze direction of a person, as far as the necessities to show a picture with said symbols is concerned to the person in question in a fixed position with respect to one of the eyes of the person and the arrangement of the means determining the gaze direction of the person looking at said picture in a fixed position with respect to one eye of the person.

By the arrangement of the picture emitting element and the light sensitive element registering an image of the eye of the person on a carrier, which is located closely in front of the eyes of the person and in a predetermined position with respect to the eye to be examined, the drawbacks associated with the devices already known were avoided, in which it was necessary that the person would hold his eye in a determined position with respect to the picture shown as well as the means determining the gaze direction so as to make the device in question able to function.

However, although a person using this already known communication device for communicating with the environment when communicating may turn his head in an arbitrary direction and accordingly is not in any way restricted in motion or position, errors may arise in the determination of towards what on the picture shown the person is directing his gaze since the carrier is displaced somewhat laterally with respect to the eye of the person. The definition lateral displacement comprises displacements in the vertical direction as well as in the horizontal direction. It will be sufficient for the person in question to contract his nose at one single occasion so as to generate such a displacement. Other face movements or displacements of the head of the person may also cause a certain lateral displacement of the carrier with respect to the eye of the person. Since the gaze direction of the eye of the person in this device is determined by detecting the position of the pupil on an image of the eye thrown upon the light sensitive element, a lateral displacement of this kind will, after an adjustment and testing of the device previously made, make the evaluating unit make incorrect conclusions as to what on the picture shown the person is holding his gaze at, so that a new adjustment with a testing run becomes necessary. Lateral displacements of one or some millimetres will be enough to cause such errors. Not only will it be necessary for a person having, for any reason, difficulties to hold his face parts still to irritatingly often readjust the device, but the person may also deliver messages leading to other results than he intended.

### BRIEF DESCRIPTION OF THE INVENTION

The object of the present invention is to provide a device of the type defined in the introduction, but which eliminates the drawbacks as discussed above of the device already known of this type and enables a reliable determination of towards what on the picture shown said person directs his gaze independently of small lateral displacements between the carrier and the eye of the person and without necessitating any readjustment after an initial adjustment and such a relative displacement.

According to the invention this object is obtained by providing a device of the type defined in the introduction, characterized in that the evaluating unit comprises means to select, from the picture information emanating from the light emitted by the eye of the person on the light sensitive element, two different picture information parameters emanating from the eye of the person, which have a mutual distance on the light sensitive element which is a function of the angle made by the gaze direction of the eye with the optical axis of the optical system and independent of possible small lateral displacements between the carrier and the eye of the person, and means to calculate said angle and thus the gaze direction of the person from said picture information parameters.

Through the understanding according to the invention that it is possible, from an image of an eye of a person generated by light reflected from this eye, to obtain two picture information parameters, the mutual distance of which is a function of the angle which the gaze direction of the eye makes with the optical axis of the optical system and independent of small lateral displacements between the carrier and the eye of the person, a device for reliable communication with the environment, controlling machines or the like without any requirement of an exact lateral fixation of the carrier of the device may be provided. According to the invention such a device is obtained by constructing the evaluating unit in the way mentioned above.

According to a preferred embodiment of the invention one of said picture information parameters consists of a light reflex emanating from the cornea of the eye and the other picture information parameter is an image of a point being arranged to follow the eye in its movement. An example of such a point is the pupil opening of the eye, the picture information parameter used in such a case being preferably one outer border of the pupil opening. The special features of the cornea sphere of the eye regarding the reflection of light inciding thereon are utilized in this preferred embodiment.

Further preferred features and advantages of the device according to the invention will appear from the other dependent claims and the appended description.

BRIEF DESCRIPTION OF THE DRAWINGS

With reference to the appended drawings, below follows a specific description of a preferred embodiment of the invention cited as an example.

In the drawings:

Fig 1 is a schematic perspective view of a person using the device according to the invention,

Fig 2 is a diagram illustrating the construction and the function of a device according to a preferred embodiment of the invention,

Figs 3-6 are views contributing to the explanation of the optical considerations on which the design of the device according to the preferred embodiment of the invention are based upon, and

Fig 7 is a simplified schematic perspective view of a part of the device according to the invention.

DETAILED DESCRIPTION OF A PREFERRED EMBODIMENT OF THE INVENTION

It should be pointed out that the invention is not in any way restricted to devices intended to be used by disabled persons, but the invention may just as well be applied to different work situations and the like in which a sound and healthy person may find himself and in which the same has his hands occupied or for any other reason wishes to use his eye for communicating with any other person, controlling a machine or the like.

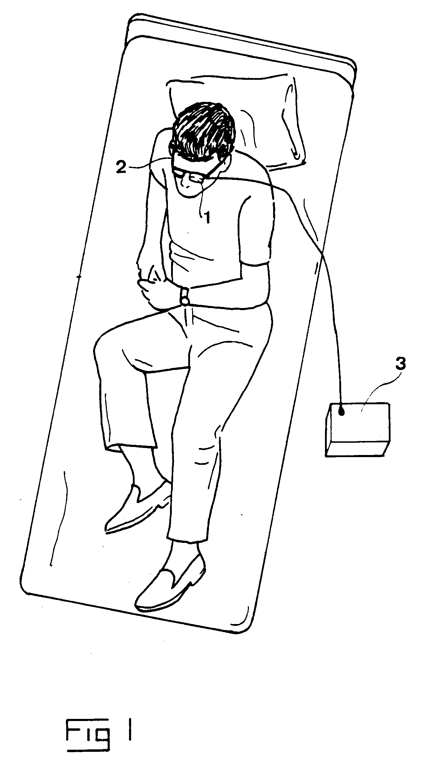

A disabled person using the communication device according to the invention is shown in Fig 1, but as mentioned the device could also be used by for example a pilot. The device has an optical unit 1, which is arranged on a, for example spectacle-frame-like, carrier 2 arranged in a fixed distance in front of the eyes of the user, so that the optical unit is arranged directly in front of one eye of the user. Leads run from the optical unit 1 to a central processing unit 3 located at an arbitrary, suitable place, which contains means for controlling different functions of the optical unit and to which further equipment is connectable.

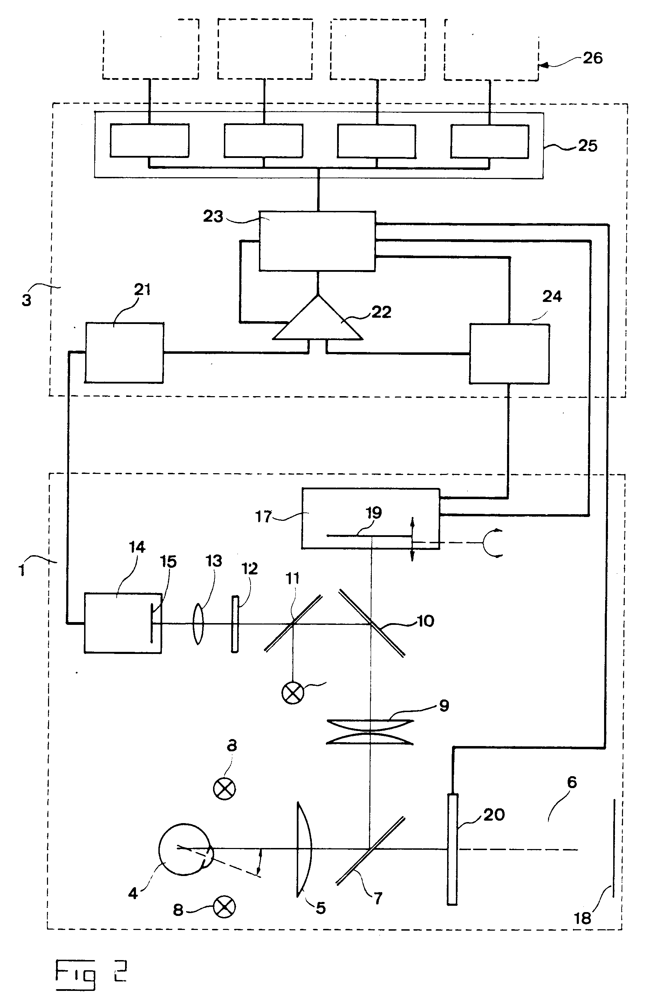

The function of the device according to the invention will now be described with reference to Fig 2, in which the optical unit 1 arranged on the carrier and the central unit 3 each has been framed in by dashed lines. One of the eyes of the person is provided with the reference 4. A spectacle lens 5 is arranged in front of the eye 4 of the person for correcting possible visual defects. The eye 4 looks at the environment 6 through a first plane mirror 7 semitransparent to the visible light. The field of view is thereby restricted to 30° horizontally and vertically. Two light sources 8 emitting infrared light, in this case in the form of a light emitting diode, the emitted light of which is led by a thin light fibre bundle to a small prism which finally reflects towards the eye, are arranged beside the eye 4 of the person to illuminate the eye 4 obliquely from the sides so as to achieve a good contrast between the dark pupil and the surrounding iris on the image of the eye thrown upon the first plane mirror 7. A system of lenses 9 acting as a convex lens is arranged along an optical axis extending through the rotational centre of the eye and in reflexion by the plane mirror 7 in such a way, that the pupil of the eye lies substantially in the focal plane of the system of lenses, which means that light beams emitted from one point of the pupil of the eye and reflected by the first plane mirror 7 towards the system of lenses 9 will be refracted by the system of lenses 9, so that they leave this in the form of parallel light beams. The first plane mirror 7 is preferably high-reflecting just for infrared light.

A second plane mirror 10 also semitransparent to visible light and high-reflecting to infrared radiation is arranged along the optical axis passing through the system of lenses 9. The light beams emanating from the eye 4 and refracted by the system of lenses 9 are reflected by the second plane mirror 10 towards a third semitransparent plane mirror 11, through which the main part of the light beams pass, and through a band-pass filter 12, which is arranged to filter out the main part of the light not being in the wave-length field of the beams emanating from the light sources 8 - in this case about 880 nm. The parallel light beams emanating from the eye then arrive to the objective 13, in the form of a convex lens, of a video camera 14. The video camera 14 has a detector plane 15 located in the focal plane of the objective 13, on which the beams emanating from one point of the eye and made parallel by the system of lenses are refracted by the objective 13 together in one point. An image of the eye 4 is in this way created on the detector plane 15 and thanks to the light sources 8 there is a good contrast in this image between the dark pupil and the surrounding iris.

The optical unit 1 also comprises a further light source 16 emitting infrared light of the same wavelength as the light sources 8. This light source 16 consists of a light emitting diode and is located in the focal plane of the system of lenses 9 and is directed so that the light emitted therefrom firstly will be reflected by the third plane mirror and then by the second plane mirror against the system of lenses 9 along the optical axis of the optical system. This means that the light beams emitted by the light source 16 will be refracted by the optical system of lenses into parallel light beams, which are reflected by the plane mirror 7 towards the cornea of the the eye. The cornea of the eye functions in such a way that it reflects a light beam inciding thereupon as if it came from a point on the half radius of curvature of the cornea, and by the fact that such a point lies in substantially the same plane as the pupil and thereby in the focal plane in the system of lenses 9 the light beams emanating from the light source 16 and reflected by the cornea of the eye will after repeated reflexion by the first plane mirror be refracted by the system of lenses 9 to parallel beams, which are reflected by the second plane mirror 10, pass through the third plane mirror 11 and the band-pass filter 12 and finally are refracted by the objective 13 into an image of the cornea reflex on the detector plane 15 of the video camera 14. It will be explained later on that the gaze direction of the eye may be determined from the pieces of information about the position of the cornea reflex and the pupil on the detector plane 15.

A video monitor 17 is arranged in a fixed position on the carrier and with respect to the other optical elements just described. The video monitor 17 is arranged to show pictures with different symbols, which the person may hold his gaze at so as to communicate with the environment, control the video monitor or any machine or the like, to the eye 4 of the person. The video monitor 17 emits picture light passing through the second plane mirror 10 and the system of lenses 9 and is then reflected by the first plane mirror 7 towards the eye 4 of the person, so that a visible virtual picture 18 is obtained behind the plane mirror 7. The virtual picture 18 is located at a fixed distance with respect to the eye 4 of the person, but this distance may be variated by adjusting the display plane 19 of the video monitor 17. An electronic light limiter 20 is arranged behind the plane mirror 7 to regulate the extent of light from the environment 6. The electronic light limiter 20 may be controlled by the person by looking through the eye 4 at a suitable symbol on the virtual picture 18 displayed by the video monitor. The virtual picture is in reality of course located at a considerable distance in front of the optical unit, namely between 30 cm and at an indefinite sight distance therefrom.

Picture signals emanating from the detector plane of the video camera 14 are led to a picture evaluating unit 21 included in the central processing unit 3 and arranged to evaluate these picture signals and from them and other information about the parameters in the optical system previously described calculate the gaze direction of the eye 4 of the person. Then the evaluating unit 21 sends information about at which point on the virtual picture the person is looking onto a fixation unit 22, which is arranged to determine whether the gaze direction of the user corresponds to any command, i.e. whether the gaze direction lies within or without the hit region centered around each fixation point. The fixation unit 22 also decides if the eye is completely still or roving and whether the user has fixed the gaze for a time long enough to make a command considered as selected. If all conditions are met with the fixation unit 22 will send a signal to a command system 23, which in principle is a computer program package with different command procedures, which in the practice define what may be carried out with the product. Furthermore the carrying out of a command is indicated by immediate light signalization and/or by visual indication adjacent to the symbol in question on the virtual picture through a menu system 24. The command system 23 is arranged to send control signals to the menu system 24, which in its turn controls the video monitor 17 to display different menus to the eye 4 of the person. Furthermore, the command system 23 is also adapted to send control parameters to the fixation unit 22. The electronic light limiter 20 may also be controlled through the command system 23. This whole controlling may be carried out by the person in question by looking at different symbols or fields of the virtual picture 18 shown by the video monitor 17. The command system 23 also contains the routines 25, which enable two-way communication with equipments 26, which are connected to the central processing unit 3. The equipments 26 may comprise a pointing-out screen, an alarm and calling apparatus, optional accessories and the like.

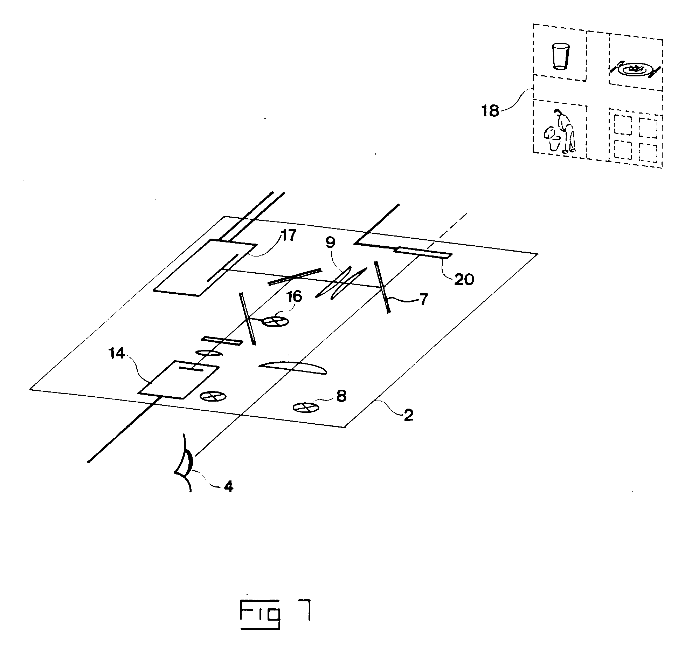

The function of the device, which can be understood by means of the abovementioned, is very diagrammatically illustrated in the appended Fig 7.

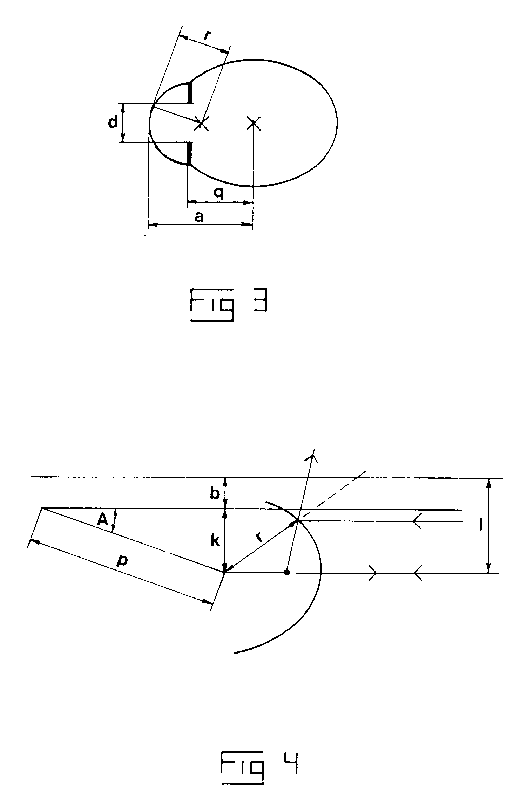

The purpose of the device according to the invention is to determine accurately towards which point on the virtual picture 18 the gaze of the eye 4 of the person is directed also when the carrier 2, i.e. the optical unit 1, is slightly laterally displaced with respect to the eye 4 of the person. The way to obtain this and the considerations and calculations behind this will be explained below with reference to Figs 3-6 also.

When the user is studying a fixation point on the virtual picture the eye 4 is turned by an angle A with respect to the optical axis. In order to decide on which of the fixation points of the virtual picture the eye is fixed to, the angle of rotation A of the eye must be determined. It will now be explained how this may be achieved in an accurate manner despite a lateral displacement of the optical unit with respect to the eye.

The parallel light emanating from the light source 16 and inciding towards the eye is partially reflected by the cornea of the eye, which acts as a convex, spherical mirror with the radius of curvature r. The beam intersecting the centre of the cornea globus is reflected directly in the opposite direction, while the other beams are reflected in varying directions, and for small angles it is true that they are reflected as if they came from a point at a distance r/2 from the front edge of the cornea. Such points at the distance r/2 from the front edge of the cornea lie in substantially the same plane being perpendicular to the optical axis of the optical system and coinciding with the focal plane of the system of lenses 9.

The lateral displacement of the light reflex as a function of the distortion of the eye, i.e. the angle, is the same as the lateral displacement of the centre of the cornea sphere, i.e.

$$k = p \sin A \qquad (1)$$

in which p is the distance between the rotation centre of the eye and the centre of the cornea sphere. Furthermore, it appears as when the eye is laterally displaced with respect to the optical axis the total displacement of the reflex will be

$$l = b + p \sin A \qquad (2)$$

A point-like light source at the distance u from the optical axis gives rise to a parallel beam bundle inciding with the angle C, given by the relation

$$u/f = \tan C \qquad (3)$$

in which f is the focal distance of the system of lenses. The distance of the reflex, e, from the optical axis will then be

$$e = (r/2) \tan C \qquad (4)$$

after inserting (3) in (4) the enlarging factor of the image will be

$$K = e/u = r/(2f) \qquad (5)$$

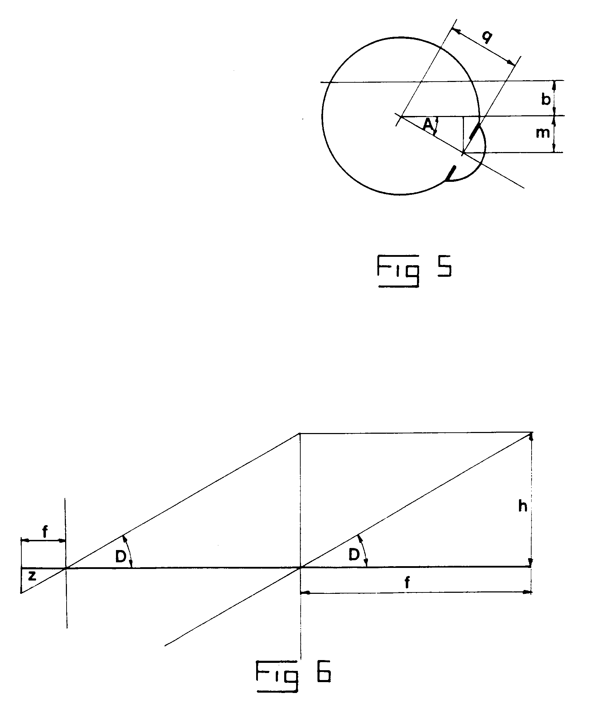

The pupil pu is located at the distance q from the rotation centre of the eye (see Fig 5). The displacement of the pupil centre from the optical axis as a function of the distortion A of the eye will be

$$m = q \sin A \qquad (6)$$

If the eye is laterally displaced by the distance b with respect to the optical axis the total displace-ment will instead be

$$n = b + q \sin A \qquad (7)$$

Reference is now also made to Fig 6. The eye located in the focal plane of the system of lenses emits beams from a picture point at the distance h from the optical axis and creates a parallel beam bundle with the angle D out of the system of lenses given by the relationship

$$h/f = \tan D \qquad (8)$$

The beam bundle is refracted together by the lens of the camera objective to a point z in the detector plane, which is given by the relationship

$$z/f_{obj} = \tan D \qquad (9)$$

in which $f_{obj}$ is the focal distance of the objective. From this follows that the enlarging factor L of the camera image will be

$$L = z/h = f_{obj}/f \qquad (10)$$

The total enlarging factor M for the image of the reflex lightening will through multiplication by (5) be

$$M = KL = (rf_{obj})/(2f^2) \qquad (11)$$

the relation (1) and (6) give for the distortion of the reflex and pupil centre

$$z(rc) = Lp \sin A \qquad (12)$$

and

$$z(pc) = Lq \sin A \qquad (13)$$

Furthermore, the size of the pupil and reflex illumination, d and u, respectively are given by

$$z(rs) = M2u \qquad (14)$$

and

$$z(ps) = Ld \qquad (15)$$

From (2) and (7) it is noted that the difference between the distortion of the pupil and the reflex will be independent of the lateral displacement l, so

that

$$z(prc) = z(ps) - z(rs) = L (q-p) \sin A \qquad (16)$$

The evaluating unit 21 calculates by means of this equation the angle A and thus the distortion of the eye. L is obtained through the equation (7), Z(prc) is measured by the detector plane 15 of the video camera and q and p are constant parameters stored in the picture evaluating unit. Thus, it appears that the difference between the distortion of the pupil and the reflex will be totally independent of the lateral displacement of the optical unit with respect to the eye. The device may thanks to this fact deliver reliable results even if the optical unit is displaced somewhat with respect to the eye. The evaluating unit will still be able to reliable calculation of the distortion of the eye and thus towards which point on the picture displayed by the video monitor the gaze of the eye is directed.

In the case in question f = 80 mm, $f_{obj}$ = 16 mm, q = 10.1 mm and p = 5.3 mm. This results in L = 0.20 and M = 0.010, so that for the distortion A = 20°: = 16 mm, q = 10.1 mm and p = 5.3 mm. This results in L = 0.20 and M = 0.010, so that for the distortion A = 20°:

z(rc) = 0.36 mm or about 18 micrometers/degree
z(pc) = 0.69 mm or about 35 micrometers/degree
z(rs) = 0.03 mm (u = 1.5 mm)
z(ps) = 1.6 mm (d = 8 mm)
z(prc) = 0.33 mm or about 17 micrometers/degree

The detector used has the following data.

Detector surface = 6.0 x 4.5 mm
size of the picture element = 10 x 15.6 micrometers
vertical resolution = 288 lines
horizontal resolution = 600 picture points.

288 lines in 4.5 mm correspond to about 15.6 micrometers/line.

At the distortion A = 20° and the maximum pupil diameter the outer edge of the pupil lands at the distance 0.69 + 1.6/2 mm = 1.49 mm from the centre point of the picture element. The distance to the picture border will then be 4.5/2-1.49 = 0.76 mm, which results in a maximally allowed decentering, i.e. lateral displacement, of the eye in the eye plane of about 0.76/0.2 = 3.8 mm. This has turned out to be quite sufficient.

Thus, the invention is based on the understanding that information may be obtained from an image of the eye being in a connection, which is independent of a lateral displacement of the optical unit with respect to the eye, and that these pieces of information may be used to calculate the distortion or angle of the eye, so that the result of such a calculation is independent of possible lateral displacements of said kind. In order to achieve this the particular properties of the cornea of the eye and particular arrangement of different components of the optical system with respect to each other and with respect to the eye are used, so that light emitted, or more accurate reflected, by the cornea as well as by the pupil will substantially emanate from the focal plane of the system of lenses.

It would for example be conceivable to choose any other point following the eye in its motion than the pupil. A contact lens provided with a mark may for instance be placed on the eye of the person and the position of this mark in the detector plane could be used instead of the position of the pupil.

## Claims

1. A device for making it possible for in particular disabled persons without capability to speak and to move their arms to communicate with the environment, comprising means (7-15) adapted to detect the direction of the gaze of one of the eyes (4) of said person, a unit (21) adapted to evaluate the direction information from said means so as to deliver a signal, the character of which depends on the direction of the gaze of said person, an apparatus (17, 24) arranged to show one or more pictures to the person and having a picture emitting element (17), said pictures having symbols or text corresponding to different information, which the person may be expected to wish to communicate to for instance another person or a machine, said evaluating unit being adapted to calculate towards what on said picture the person is directing his gaze, said detecting means comprising a light sensitive element (14) towards which the light emitted by one of the eyes of the person is intended to directly or indirectly fall, said detecting means also comprising an optical system (7, 9-11) arranged closely in front of one of the eyes of said person and in a fixed position with respect thereto, said system comprising mirrors (7, 10, 11) and/or lenses (9) and is arranged to throw an image of the eye of the person directly upon said light sensitive element, said evaluating unit being arranged to calculate the direction of the gaze of said eye of the person through information from the image of the eye of the light sensitive element, the device comprising a carrier (2), for example in the form of a spectacle-frame-like element, at least said picture emitting element (17) of the apparatus and the optical system of the detecting means and also the light sensitive element being arranged on the carrier at mutually fixed distances, said carrier being intended to be located closely in front of the eyes of the person and in a predetermined position with respect to the eye to be examined, **characterized** in that the evaluating unit (21) comprises means to select, from the picture information emanating from the light emitted by the eye of the person on the light sensitive element (14, 15), two different picture information parameters emanating from the eye of the person, which have a mutual distance on the light sensitive element which is a function of the angle (A) made by the gaze direction of the eye with the optical axis of the optical system and independent of possible small lateral displacements between the carrier and the eye of the person, and means to calculate said angle and thus the gaze direction of the person from said picture information parameters.

2. A device according to claim 1, **characterized** in that one of said picture information parameters is a light reflex emanating from the cornea of the eye and the other picture information parameter is an image of a point arranged to follow the eye in the motion thereof.

3. A device according to claim 2, **characterized** in that said point following the eye is the pupil opening (pu) of the eye.

4. A device according to claim 2 or 3, **characterized** in that the optical system comprises a system (9) of one or several lenses acting as a convex lens, that said means comprises a light source (16) located in the focus of said system of lenses on a first side thereof, and that the optical system is adapted to throw the parallel light beams emanating from the light source and emerging on the other side of the system of lenses upon the eye of the person.

5. A device according to claim 4, **characterized** in that the system of lenses (9) is so located on the carrier that the centre of the cornea sphere of the eye (4) of the person is in a distance being substantially half of the radius of the curvature (r) of the cornea behind the focal plane of the system of lenses on said second side, so that the parallel light beams from the light source inciding towards the eye and reflected by the cornea as if they were emanating from one point on the half of the radius of curvature of the latter will be refracted by the system of lenses (9), so that they become parallel to each other when emerging on its first side.

6. A device according to claim 5, **characterized** in that the light sensitive element comprises a camera (14) and that the objective (13) of the camera is located on said first side of the system of lenses with its optical centre on the optical axis of the system of lenses, so that the parallel light beams emanating from the cornea reflex are refracted together to a point in the focal plane of the camera objective.

7. A device according to any of the claims 4-6, **characterized** in that the picture emitting element (17) of the apparatus is arranged along the optical axis of the system of lenses on the first side thereof, and that the picture emitting element is arranged to show a picture by emitting it towards the system of lenses (9) along the optical axis thereof.

8. A device according to claim 1, **characterized** in that the optical system comprises a first plane mirror (7), intended to be located in a predetermined position in front of the eye (4) of the person, and that said mirror is arranged to, through other elements included in the optical system, create a virtual image shown by the apparatus behind the first plane mirror.

9. A device according to claims 4 and 8, **characterized** in that said first plane mirror (7) is arranged to reflect light beams coming from the system of lenses (9) towards the eye (4) of the person and light beams reflected by the eye of the person towards the system of lenses.

10. A device according to claim 9, **characterized** in that the optical system comprises a second plane mirror (10) arranged to reflect light beams refracted by the system of lenses (9) and emanating from the eye towards the light sensitive element, and that this plane mirror is semitransparent and located in the path taken by the picture of the picture emitting element towards the system of lenses.

11. A device according to claim 10, **characterized** in that the optical system comprises a third plane mirror (11) located between the second plane mirror (10) and the light sensitive element (14, 15), that this plane mirror is semitransparent for letting light beams reflected by the second plane mirror towards the light sensitive element through, and that said third plane mirror is arranged to reflect light emitted by said light source (16) towards the second plane mirror (10) so that it hereby will be reflected towards the system of lenses (9).

**Patentansprüche**

1.  Vorrichtung, die es ermöglicht besonders behinderten Personen, ohne die Fähigkeit zu sprechen oder ihre Arme zu bewegen, mit ihrer Umgebung zu kommunizieren, die umfaßt: eine Einrichtung (7-15), die geeignet ist, die Blickrichtung eines der Augen (4) dieser Person zu erfassen, eine Einheit (21), die geeignet ist, die Richtungsinformation dieser Einrichtung auszuwerten und ein Signal abzugeben, dessen Eigenschaften von der Blickrichtung dieser Person abhängen, eine Vorrichtung (17, 24), die eingerichtet ist, der Person eines oder mehrere Bilder zu zeigen und die ein bildaussendendes Element (17) aufweist, wobei diese Bilder mit Symbolen oder Text versehen sind, die unterschiedlichen Informationen entsprechen, von denen angenommen wird, daß die Person sie beispielsweise einer anderen Person oder einer Maschine mitteilen will, wobei diese Auswerteeinheit geeignet ist zu ermitteln, worauf in diesem Bild die Person ihre Blickrichtung gerichtet hat, und wobei diese Erfassungseinrichtung ein lichtempfindliches Element (14) aufweist, auf das das von einem Auge dieser Person reflektierte Licht direkt oder indirekt fallen soll, wobei diese Erfassungseinrichtung außerdem ein optisches System (7, 9-11) umfaßt, das nahe vor einem der Augen dieser Person in einer festen Position bezüglich dieses Auges angeordnet ist, wobei dieses System Spiegel (7, 10, 11) und/oder Linsen (9) umfaßt und geeignet ist, ein Bild des Auges dieser Person direkt auf dieses lichtempfindliche Element zu werfen, wobei diese Auswerteeinheit angeordnet ist, um die Blickrichtung dieses Auges der Person aus Information von dem auf das lichtempfindliche Element geworfenen Bild des Auges zu errechnen, wobei diese Einrichtung einen Träger (2) umfaßt, etwa in Form eines Elements ähnlich einem Brillenrahmen, und wenigstens das bildaussendende Element (17) der Vorrichtung und das optische System der Erfassungseinrichtung und auch das lichtempfindliche Element auf diesem Träger in gegenseitigem festen Abstand angeordnet sind, wobei beabsichtigt ist, den Träger nahe vor den Augen dieser Person in einer vorher festgelegten Position relativ zu dem zu untersuchenden Auge anzubringen, **dadurch gekennzeichnet**, daß die Auswerteeinheit (21) eine Einrichtung umfaßt, um aus der Bildinformation, die aus dem von dem Auge der Person auf das lichtempfindliche Element (14, 15) ausgesandten Licht hervorgeht, zwei unterschiedliche Parameter der Bildinformation von dem Auge der Person auszuwählen, die einen gegenseitigen Abstand auf dem lichtempfindlichen Element aufweisen, der eine Funktion des Winkels (A) ist, der zwischen der Blickrichtung des Auges und der optischen Achse des Systems gebildet wird und unabhängig ist von kleinen seitlichen Abweichungen zwischen dem Träger und dem Auge der Person, und die Auswerteeinheit (21) eine Einrichtung zum Errechnen dieses Winkels und damit zur Blickrichtung der Person aus diesen Parametern der Bildinformation aufweist.

2.  Vorrichtung nach Anspruch 1, **dadurch gekennzeichnet**, daß einer dieser Parameter der Bildinformation ein Lichtreflex von der Hornhaut des Auges ist und der anderer dieser Parameter der Bildinformation das Bild eines Punktes ist, der dem Auge bei dessen Bewegung folgt.

3.  Vorrichtung nach Anspruch 2, **dadurch gekennzeichnet**, daß der dem Auge folgende Punkt die Pupillenöffnung (pu) des Auges ist.

4.  Vorrichtung nach Anspruch 2 oder 3, **dadurch gekennzeichnet**, daß das optische System ein System (9) von einer oder mehreren Linsen umfaßt, die sich wie eine konvexe Linse verhalten, daß diese Einrichtung eine Lichtquelle (16) umfaßt, die im Brennpunkt dieses Linsensystems auf einer ersten Seite davon angeordnet ist und daß das optische System geeignet ist, die parallelen Lichtstrahlen, die von der Lichtquelle ausgehen und auf der anderen Seite des Linsensystems austreten, auf das Auge der Person zu werfen.

5.  Vorrichtung nach Anspruch 4, **dadurch gekennzeichnet**, daß das Linsensystem (9) so auf dem Träger angeordnet ist, daß die Mitte des Hornhautbereichs des Auges (4) in einem Abstand von im wesentlichen des halben Krümmungsradius' der Hornhaut hinter der Brennebene des Linsensystems auf dieser zweiten Seite angeordnet ist, so daß die parallelen Lichtstrahlen der Lichtquelle, die auf das Auge fallen und von der Hornhaut reflektiert werden, als ob sie von einem Punkt auf der Hälfte des Krümmungsradius' der Hornhaut kämen, von dem Linsensystem (9) erneut gebrochen werden, so daß sie parallel zueinander verlaufen, wenn sie auf der ersten Seite austreten.

6.  Vorrichtung nach Anspruch 5, **dadurch gekennzeichnet**, daß das lichtempfindliche Element eine Kamera (14) umfaßt und daß das Objektiv (13) der Kamera auf dieser ersten Seite des Linsensystems angebracht ist, wobei sein optischer Mittelpunkt auf der optischen Achse des Linsensystems liegt, so daß die parallelen Lichtstrahlen, die von der Reflektion auf der Hornhaut herrühren, gebrochen werden auf einen Punkt in der Fokussierebene des Kameraobjektivs.

7. Vorrichtung nach einem der Ansprüch 4-6, **dadurch gekennzeichnet**, daß das bildaussendende Element (17) der Vorrichtung längs der optischen Achse des Linsensystems auf dessen erster Seite angebracht ist und daß das bildaussendende Element so angeordnet ist, daß es ein Bild zeigt, indem es dieses durch das Linsensystem (9) längs dessen optischer Achse aussendet.

8. Vorrichtung nach Anspruch 5, **dadurch gekennzeichnet**, daß das optische System einen ersten planen Spiegel (7) umfaßt, der in einer festgelegten Position vor dem Auge (4) der Person angebracht werden soll, und daß der Spiegel so angeordnet ist, um durch andere im optischen System enthaltene Elemente ein virtuelles, von der Vorrichtung hinter dem ersten planen Spiegel gezeigtes Bild zu erzeugen.

9. Vorrichtung nach den Ansprüchen 4 und 8, **dadurch gekennzeichnet**, daß dieser erste plane Spiegel (7) angeordnet ist, um Lichtstrahlen, die von dem Linsensystem (9) kommen, in Richtung des Auges (4) der Person zu reflektieren und Lichtstrahlen, die vom Auge der Person reflektiert werden, in Richtung des Linsensystems zu reflektieren.

10. Vorrichtung nach Anspruch 9, **dadurch gekennzeichnet**, daß das optische System einen zweiten planen Spiegel (10) umfaßt, der angeordnet ist, um Lichtstrahlen, die von dem Linsensystem gebrochen sind und von dem Auge in Richtung des lichtempfindlichen Elements ausgehen, zu reflektieren, und daß dieser plane Spiegel halbdurchlässig ist und angeordnet ist in dem Pfad, den das Bild von dem bildaussendenden Element in Richtung des Linsensystems nimmt.

11. Vorrichtung nach Anspruch 10, **dadurch gekennzeichnet**, daß das optische System einen dritten planen Spiegel (11) umfaßt, der zwischen dem zweiten planen Spiegel (10) und dem lichtempfindlichen Element (14, 15) angeordnet ist, daß dieser plane Spiegel halbdurchlässig ist, um Lichtstrahlen, die von dem zweiten planen Spiegel in Richtung des lichtempfindlichen Elements reflekltiert werden, passieren zu lassen, und daß dieser dritte plane Spiegel so angeordnet ist, um Licht, das von der Lichtquelle (16) in Richtung des zweiten planen Spiegels (10) ausgesandt wird, zu reflektieren, so daß es hierdurch in Richtung des Linsensystems (9) reflektiert wird.

**Revendications**

1. Dispositif permettant, en particulier aux handicapés qui ne sont pas capables de parler et de remuer leurs bras, de communiquer avec leur environnement, ce dispositif comportant des moyens (7-15) aptes à détecter la direction du regard d'un des yeux (4) d'une telle personne, une unité (21) apte à analyser l'information de direction fournie par lesdits moyens en vue d'émettre un signal dont le caractère dépend de la direction du regard de la personne, un appareil (17, 24) agencé pour montrer une ou plusieurs images à la personne et comprenant un élément d'émission d'images (17), lesdites images portant des symboles ou un texte qui correspond à différentes informations que la personne est supposée vouloir comuniquer par exemple à une autre personne ou à une machine, ladite unité d'analyse étant apte à calculer vers quoi sur l'image la personne dirige son regard, lesdits moyens de détection comprenant un élément photosensible (14) sur lequel la lumière émise par l'un des yeux de la personne doit tomber directement ou indirectement, lesdits moyens de détection comprenant également un système optique (7, 9-11) agencé à faible distance devant l'un des yeux de la personne, dans une position fixe par rapport à cet oeil, ledit système comprenant des miroirs (7, 10, 11) et/ou des lentilles (9) et étant agencé pour projeter une image de l'oeil de la personne directement sur ledit élément photosensible, l'unité d'analyse étant agencée pour calculer la direction du regard dudit oeil de la personne à partir d'informations de l'image de l'oeil de l'élément photosensible, le dispositif comprenant un support (2), par exemple en forme d'une monture de lunettes, au moins ledit élément (17) d'émission d'images de l'appareil et le système optique des moyens de détection, ainsi que l'élément photosensible étant disposés sur le support à des distances fixes les uns par rapport aux autres, ledit support devant être agencé à faible distance devant les yeux de la personne et dans une position prédéterminée par rapport à l'oeil scruté, caractérisé par le fait que l'unité d'analyse (21) comprend des moyens de sélection, à partir des informations de l'image émanant de la lumière émise par l'oeil de la personne sur l'élément photosensible (14, 15), de deux paramètres différents d'information de l'image émanant de l'oeil de la personne dont la distance mutuelle sur l'élément photosensible est fonction de l'angle (A) formé par la direction du regard de l'oeil avec l'axe optique du système optique et est indépendante d'éventuels déplacements latéraux faibles entre le support et l'oeil de la personne, ainsi que des moyens pour calculer ledit angle et ainsi la direction du regard de la personne à partir desdits paramètres d'information de l'image.

2. Dispositif selon la revendication 1, caractérisé en ce que l'un des paramètres d'information de l'image est une réflexion de lumière émanant de la cornée de l'oeil et l'autre paramètre d'information de l'image est l'image d'un point agencé pour suivre le mouvement de l'oeil.

3. Dispositif selon la revendication 2, caractérisé en ce que ledit point qui suit l'oeil est l'ouverture de la pupille (pu) de l'oeil.

4. Dispositif selon la revendication 2 ou 3, caractérisé en ce que le système optique comprend un système (9) comportant une ou plusieurs lentilles qui agissent en tant que lentille-(s) convexe(s), que ledit moyen comprend une source de lumière (16) agencé au point focal dudit système de lentilles sur un premier côté de celui-ci, et que le système optique est apte à projeter les faisceaux de lumière parallèles qui émanent de la source de lumière et émergent de l'autre côté du système de lentilles, sur l'oeil de la personne.

5. Dispositif selon la revendication 4, caractérise en ce que le système de lentilles (9) est disposé sur le support de manière à ce que le centre de la région de la cornée de l'oeil (4) de la personne soit à une distance sensiblement égale au demi-rayon de courbure (r) de la cornée derrière le plan focal du système de lentilles disposé sur le deuxième côté, les faisceaux de lumière parallèles de la source de lumière incident vers l'oeil et étant réfléchis par la cornée comme s'ils émanaient d'un point sur le demi-rayon de courbure de celle-ci étant ainsi réfractés par le système de lentilles (9) de manière à devenir parallèles entre eux lorsqu'ils émergent sur le premier côté.

6. Dispositif selon la revendication 5, caractérisé en ce que l'élément photosensible comprend une caméra (14) et que l'objectif (13) de la caméra est disposé sur ledit premier côté du système de lentilles, son centre optique étant situé sur l'axe optique du système de lentilles de manière à ce que les faisceaux de lumière parallèles émanant de la réflexion de cornée soient réfractés ensemble vers un point du plan focal de l'objectif de la caméra.

7. Dispositif selon l'une quelconque des revendications 4 à 6, caractérisé en ce que l'élément d'émission d'images (17) de l'appareil est disposé selon l'axe optique du système de lentilles sur le premier côté de celui-ci, et que l'élément d'émission d'images est agencé pour montrer une image en la transmettant vers le système de lentilles (9) selon l'axe optique de celui-ci.

8. Dispositif selon la revendication 1, caractérisé en ce que le système optique comprend un premier miroir plan (7), devant être disposé dans une position prédéterminée devant l'oeil (4) de la personne, et en ce que ledit miroir est apte à créer, au moyen d'autres éléments compris dans le système optique, une image virtuelle montrée par l'appareil derrière le premier miroir plan.

9. Dispositif selon les revendications 4 et 8, caractérisé en ce que le premier miror plan (7) est agencé pour réfléchir des faisceaux de lumière émanant du système de lentilles (9) vers l'oeil (4) de la personne et les faisceaux de lumière réfléchis par l'oeil de la personne, vers le système de lentilles.

10. Dispositif selon la revendication 9, caractérisé en ce que le système optique comprend un deuxième miroir plan (10) agencé pour réfléchir des faisceaux de lumière réfractés par le système de lentilles (9) et émanant de l'oeil, vers l'élément photosensible, et que ce miroir plan est semi-transparent et disposé dans le trajet emprunté par l'image de l'élément d'émission d'images vers le système de lentilles.

11. Dispositif selon la revendication 10, caractérisé en ce que le système optique comprend un troisième miroir plan (11), disposé entre le deuxième miroir plan (10) et l'élément photosensible (14, 15), que ce miroir plan est semi-transparent pour laisser passer les faisceaux de lumière réfléchis par le deuxième miroir plan vers l'élément photosensible, et que le troisième miroir plan est agencé pour réfléchir la lumière émise par la source de lumière (16) vers le deuxième miroir plan (10), la lumière étant ainsi réfléchie vers le système de lentilles (9).

Fig 1

Fig 2

Fig 3

Fig 4

14

Fig 5

Fig 6

Fig 7